# EUROPÄISCHE PATENTSCHRIFT

(11) **EP 0 415 176 B1**
(45) Veröffentlichungstag und Bekanntmachung des Hinweises auf die Patenterteilung: **17.05.1995**
(21) Anmeldenummer: 90115592.9
(22) Anmeldetag: 14.08.1990
(51) Int. Cl.: A61B 6/00, F16M 11/04

(54) **Gewichtsausgleichsvorrichtung**
Balancing device
Dispositif d'équilibrage

(30) Priorität: 25.08.1989 SE 8902831
(43) Veröffentlichungstag der Anmeldung: 06.03.1991
(73) Patentinhaber: Siemens-Elema AB, 171 95 Solna 1 (SE); SIEMENS AKTIENGESELLSCHAFT, 80333 München (DE)
(72) Erfinder: Warden, Hans, S-194 51 Upplands Väsby (SE)

(56) Entgegenhaltungen:
- DE-A- 3 312 137
- DE-C- 939 348
- US-A- 4 101 779
- US-A- 4 318 538

## Beschreibung

Die Erfindung bezieht sich auf eine Gewichtsausgleichsvorrichtung für eine zweiteilige Tragarmvorrichtung bei einem Röntgengerät mit einem ersten und einem zweiten Tragarm, bei dem das eine Ende des ersten Tragarms um eine horizontale erste Welle am Röntgengerät drehbar gelagert ist und das andere Ende über eine zweite Welle mit dem einen Ende des zweiten Tragarms, an dessen anderem Ende ein Apparat befestigt ist, drehbar verbunden ist und daß jeder Tragarm mit jeweils einem Hebelarm versehen ist, der Teil eines Parallelogrammystems ist, dessen Verbindungsknoten teils am freien Ende des Hebelarms am ersten Tragarm, teils an der zweiten Welle und teils am freien Ende des Hebelarms des zweiten Tragarms angeordnet sind, wobei der im Parallelogrammsystem übrige Verbindungsknoten durch eine Kraft beeinflußt wird, die als Gewichtsausgleich für die Tragarmvorrichtung um die erste Welle dient, so daß sich die Tragarmvorrichtung in jeder Lage im Gleichgewicht befindet, und daß die jeweiligen Längen der Hebelarme so gewählt sind, daß die Kraft, die als Gleichgewicht für die Tragarmvorrichtung dient, unabhängig von der Lage der Tragarmvorrichtung konstant ist.

In der US-PS 4 101 779 ist eine Gewichtsausgleichsvorrichtung etwa dieser Art, die für ein bodenfestes Stativ für ein zahnärztliches Röntgengerät vorgesehen ist, beschrieben. Das Stativ weist eine als Parallelogrammarmsystem aufgebaute Armvorrichtung auf, die eine Röntgenröhre trägt. An dem freien Ende des Parallelogrammarms ist ein Gegengewicht angebracht, das an einer Verlängerung des einen Hebelarmes im Parallelogrammarmsystem befestigt ist. Die Verlängerung ist mit dem Hebelarm steif verbunden, so daß das Gegengewicht sich entsprechend dem Hebelarm bzw. der Röntgenröhre in horizontaler und vertikaler Richtung bewegt. Da die Röntgenröhre bei einer zahnärztlichen Untersuchung nur geringfügig bewegt werden muß, ist eine Gewichtsausgleichsvorrichtung dieser Art bei einem solchen Röntgengerät ausreichend. Ansonsten würde das Gegengewicht bei größeren Bewegungen der Röntgenröhre viel Bewegungsraum brauchen.

Eine weitere Gewichtsausgleichsvorrichtung für ein Röntgengerät ist durch die DE-OS 27 04 555 bekannt, bei der jeder Tragarm mit Hilfe von jeweils einer Rolle, die mittels Federn gegen jeweils eine Kurvenscheibe drückt, ausbalanciert wird. Die Kraft jeder Rolle ist im Verhältnis zum Rotationszentrum der jeweiligen Kurvenscheibe so gerichtet, daß ein Drehmoment an den Kurvenscheiben entsteht, das dazu dient, die auf die Tragarme wirkenden Drehmomente in jeder Armlage im Gleichgewicht zu halten. Als Bewegungsübertragungsmittel zwischen den Kurvenscheiben und den jeweiligen Wellen der Tragarme werden Zahnräder und Ketten verwendet.

Der Erfindung liegt die Aufgabe zugrunde, eine Gewichtsausgleichsvorrichtung der eingangs genannten Art zu schaffen, die in ihrem konstruktiven Aufbau leicht, einfach und dadurch verhältnismäßig billig ist und bei der das Gegengewicht auch bei größeren Bewegungen der Röntgenröhre wenig Bewegungsraum benötigt.

Diese Aufgabe ist erfindungemäß, durch eine Feder gelöst, deren Kraft ein keilförmiges Teil beeinflußt, welches mit dem übrigen Verbindungsknoten verbunden und hauptsächlich in vertikaler Richtung bewegbar ist und daß die Oberfläche des keilförmigen Teiles, das durch die Federkraft beeinflußt wird, eine derartige Neigung aufweist, daß eine Teilkraft entsprechend der konstanten Kraft entsteht. Hierdurch ist nur eine Feder erforderlich, die ein keilförmiges Teil beeinflußt, um die auf die beiden Tragarme wirkenden Drehmomente in jeder Armlage im Gleichgewicht zu halten. Durch diese Konstruktion wird auch der Vorteil erreicht, daß das Gerät, das an dem einen Ende des zweiten Tragarms befestigt ist, in einer horizontalen Ebene verschoben werden kann, ohne daß sich das keilförmige Teil nennenswert in vertikaler Richtung bewegt. Bei einem derartigen Bewegungsablauf wird die Feder nicht bewegt, was geringere Reibungsverluste und geringere Abnutzung bzw. Ermüdung der Feder bedeutet.

Im Hinblick auf eine günstige konstruktive Ausgestaltung der Erfindung wird vorgeschlagen, daß das keilformige Teil mit dem übrigen Verbindungsknoten über eine Strebe verbunden ist.

In einer weiteren vorteilhaften Ausbildung der Erfindung wird vorgeschlagen, daß eine zweiteilige Gelenkarmanordnung vorgesehen ist, bei der das freie Ende des einen Arms mit der Strebe schwenkbar verbunden ist und bei der das freie Ende des zweiten Arms mit der Strebe in deren axialen Richtung verschiebbar verbunden ist und bei der die Gelenkachse zwischen den Armen in einem mit dem Röntgengerät fest verbundenen Teil derart gelagert ist, daß die Gelenkachse lediglich in einer hauptsachlich vertikalen Ebene verschiebbar ist, wobei das freie Ende des zweiten Arms durch eine Kraft in der axialen Richtung der Strebe gegen das freie Ende des ersten Arms beeinflußt wird. Hierdurch wird eine Kraftkompensierungsanordnung erhalten, die die Seitenkrafte, der die Strebe bei bestimmten Einstellungen der Tragarms ausgesetzt wird, kompensiert.

Weitere Vorteile und Einzelheiten ergeben sich aus den Unteransprüchen.

Die Erfindung ist nachfolgend anhand eines in den Zeichnungen dargestellten Ausführungsbeispiels naher erläutert. Es zeigen:
- FIG 1: eine Seitenansicht eines mobilen Röntgengerätes mit einer Gewichtsausgleichsvorrichtung nach der Erfindung und
- FIG 2: eine Kraftkompensierungsanordnung für die Gewichtsausgleichsvorrichtung nach der Erfindung.

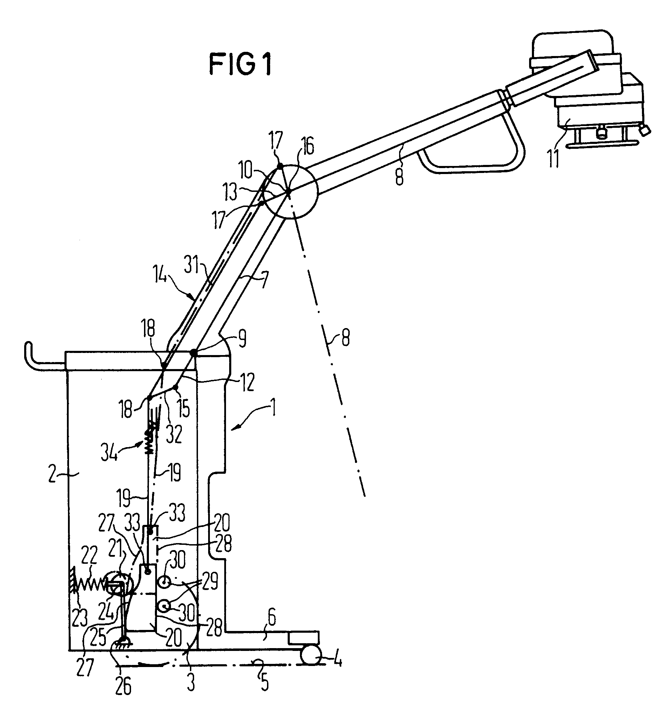

In der FIG 1 ist ein mobiles Röntgengerät (1) gezeigt mit einem Wagen (2), der mit zwei Laufrädern (3) und zwei Lenkrädern (4) versehen ist und der auf dem Boden (5) fahrbar ist. Die Lenkräder (4) sind an einem vom Wagen (2) ausgehenden Ausleger (6) befestigt. Das Röntgengerat (1) ist mit einer zweiteiligen Tragarmvorrichtung (7,8) mit einem ersten und einem zweiten Tragarm versehen. Das eine Ende des ersten Tragarms (7) ist um eine horizontale erste Welle (9) drehbar gelagert, die an der Oberseite des Wagens (2) befestigt ist. Das zweite Ende des ersten Tragarms (7) ist um eine zweite Welle (10) mit dem einen Ende des zweiten Tragarms (8) drehbar verbunden, an dessen anderem Ende eine Röntgenröhre (11) befestigt ist. Die Tragarme (7,8) sind mit jeweils einem Hebelarm (12,13) versehen, die ein Teil des Parallelogrammsystems (14) bilden, dessen Verbindungsknoten (15,16,17,18) teils am freien Ende des Hebelarms (12) am ersten Tragarm (7), teils an der zweiten Welle (10) und teils am freien Ende des Hebelarms (13) des zweiten Tragarms (8) angeordnet sind. Der übrige Verbindungsknoten (18) im Parallelogrammsystem ist über eine Strebe (19) mit einer keilförmigen Kurvenscheibe (20) verbunden. Eine Rolle (21) drückt aufgrund der Kraft einer Feder (22), die zwischen einem Befestigungspunkt (23) am Wagen (2) und einer Welle (24) der Rolle (21) eingespannt ist, gegen eine Oberflache (27) der Kurvenscheibe (20). Die Welle (24) der Rolle (21) ist außerdem über eine im wesentlichen senkrecht gegenüber der Längsrichtung der Feder (22) angebrachten Stange (25) mit einem weiteren Befestigungspunkt (26) am Wagen (2) gelenkig verbunden. Auf diese Weise druckt die Rolle (21) mittels der Feder (22) hauptsachlich in horizontaler Richtung gegen die Oberfläche (27) der Kurvenscheibe (20).

An der entgegengesetzten Oberfläche (28) der keilformigen Kurvenscheibe (20) liegen zwei Rollen (29) an, die um ihre Wellen (30) drehbar sind und die als Stütze für die Kurvenscheibe (20) dienen, so daß diese hauptsachlich in vertikaler Richtung bewegbar ist.

Die jeweiligen Längen der Hebelarme (12,13) sind so gewählt, daß die Kraft, die als Gewichtsausgleich für die Tragarmanordnung (7,8) dient, unabhängig von der Lage der Tragarmvorrichtung (7,8) konstant ist. Die Länge des Hebelarms (13) des zweiten Tragarms (8) wird so gewahlt, daß die konstante Kraft das Eigengewicht des Tragarms (8) sowie das Gewicht der Röntgenröhre (11) um die Welle (10) ausbalanciert. Die Länge des Hebelarms (12) des ersten Tragarms (7) wird so gewahlt, daß die konstante Kraft als Gewichtsausgleich für das Eigengewicht des ersten Tragarms (7) sowie für das Gewicht des zweiten Tragarms (8) und für das Gewicht der Röntgenrohre (11) um die Welle (9) dient.

Die bereits beschriebene keilförmige Kurvenscheibe (20) und die Rolle (21), die mittels der Feder (22) die Oberfläche (27) der Kurvenscheibe (20) beeinflußt, erzeugt die konstante Kraft, die erforderlich ist, um die Tragarmanordnung (7,8) mit der Röntgenröhre (11) auszubalancieren. Die Neigung entlang der Oberflache (27) weist dabei eine derartige Kurve auf, daß eine Teilkraft entsprechend der konstanten Kraft entsteht, unabhängig von der vertikalen Lage der Kurvenscheibe (20) relativ zur Rolle (21) innerhalb bestimmter Grenzen, welche in der FIG als eine obere und eine untere Grenze dargestellt sind. Die etwaige obere Grenze der Kurvenscheibe (20) relativ zur Rolle (21) wird dargestellt, indem die Kurvenscheibe (20) mit durchgezogenen Linien gezeichnet ist und die untere Grenze wird dargestellt, indem die Kurvenscheibe (20) mit strich-punktierten Linien gezeichnet ist. Die Kurve der Oberfläche (27) im Anschluß an die obere Grenze ist so gewahlt, daß die erwähnte Teilkraft geringer ist als die konstante Kraft. Wenn die Kurvenscheibe (20) in diese Lage gebracht wird, d.h., wenn die Oberfläche (27) am schmalen Teil der Kurvenscheibe (20) gegen die Rolle (21) anliegt, befindet sich die Kurvenscheibe (20) in ihrer untersten Lage.

Die Lage der keilförmigen Kurvenscheibe (20) ist von der Lage der Tragarme (7,8) abhängig, die ihrerseits das Parallelogrammsystem beeinflussen, das im Ausführungsbeispiel eine Parallelogrammarmanordnung ist, das aus dem ersten Tragarm (7) mit seinem Hebelarm (12), die gemeinsam eine erste Längsstrebe bilden, aus dem Hebelarm (13) des zweiten Tragarms (8), der eine erste Querstrebe bildet sowie aus einer zweiten Längsstrebe (31) und einer zweiten Querstrebe (32) bestehen. Wenn die Röntgenröhre (11) nach unten bewegt wird, so daß der zweite Tragarm (8) eine Lage einnimmt, die mit strich-punktierten Linien dargestellt ist, wird die Parallelogrammarmanordnung (7,12,13,31,32) um ihre Verbindungsknoten (15 bis 18) in eine Lage gedreht, die auch mit strich-punktierten Linien dargestellt ist. Hierdurch zieht der Verbindungsknoten (18) mittels der Strebe (19) die Kurvenscheibe (20) nach oben, so daß sie eine Lage einnimmt, die strich-punktiert gezeigt wird. Wenn die Röntgenröhre (11) nach oben bewegt wird, wird die Kurvenscheibe (20) nach unten in eine Lage gefahren, in der die Rolle (21) gegen die Oberfläche (27) der Kurvenscheibe in deren schmaleren Teil anliegt. In dieser Lage stellt die Kurvenscheibe (20) einen automatischen oberen Endlagestopp für die Röntgenröhre (11) dar. Diese Lage kann ein idealer Fokus-Filmabstand sein, in den die Röntgenrohre (11) rasch hineingefahren werden kann.

Wenn die Röntgenröhre (11) nach oben verschoben wird, bewegt sich die Kurvenscheibe (20) abhängig von den Bewegungen der Tragarme (7,8) um die Wellen (9) und (10) entsprechend viel in vertikaler Richtung. Wenn die Röntgenröhre (11) mittels den Tragarmen (7,8) in einer horizontalen Ebene bewegt wird, verschiebt sich die Kurvenscheibe (20) nicht oder vernachlässig wenig, was bedeutet, daß die Feder (22) bei einer solchen Bewegung nicht beeinflußt wird, was deren Lebensdauer erhöht.

In dieser FIG wird auch gezeigt, daß die Strebe (19) sowohl mit der Kurvenscheibe (20) als auch mit dem Verbindungsknoten (18) drehbar verbunden ist. Die Strebe (19) dreht sich um eine Welle (33), wenn der Verbindungsknoten (18) seitlich verschoben wird, d.h., wenn die Tragarme (7,8) bzw. die Röntgenrohre (11) bewegt wird. Um die Seitenkrafte auszugleichen, der die Strebe (19) ausgesetzt wird, wenn sie um die Welle (33) gedreht wird und die gegen die Drehung wirken, ist die Strebe (19) mit einer Kraftkompensierungsanordnung (34) verbunden, die die erwähnten Seitenkrafte ausgleicht.

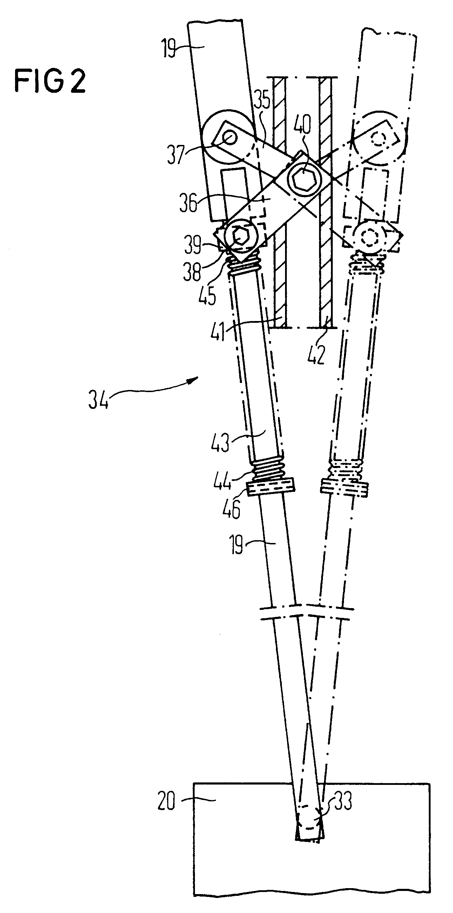

Die Kraftkompensierungsanordnung (34), die in der FIG 2 gezeigt ist, weist eine zweiteilige Gelenkarmanordnung (35,36) auf, bei der das freie Ende des einen Arms (35) mit der Strebe (19) um eine Welle (37) drehbar verbunden ist. Das freie Ende des zweiten Arms (36) ist über eine Welle (38) an einer an der Strebe (19) angebrachten Hulse (39) drehbar befestigt, die in der axialen Richtung der Strebe (19) verschiebbar gelagert ist. Die gemeinsame Gelenkachse (40) für die Arme (35) und (36) ist zwischen zwei Schienen (41,42) gelagert, die parallel miteinander verlaufen und vertikal angeordnet und mit dem Wagen (2) starr verbunden sind. Der zweite Arm (36) wird von einer Druckfeder (43) beeinflußt, deren eines Ende (44) gegen eine Hülse (46), die mit der Strebe (19) fest verbunden ist, anliegt und deren anderes Ende (45) über die Hulse (39) mit dem freien Ende des zweiten Arms (36) verbunden ist. Wegen der Druckfeder (43) wird das freie Ende des zweiten Arms (36) über die Hülse (39) durch eine Kraft in axialer Richtung der Strebe (19) in Richtung auf das freie Ende des ersten Arms (35) beeinflußt. Wenn die Strebe (19) eine in der FIG 2 dargestellte Lage eingenommen hat, wird die Strebe (19) mit Hilfe der Kraftkompensierungsanordnung (34) am Platz gehalten. Die Seitenkraft, der die Strebe (19) in ihrer Schräglage ausgesetzt wird, wird durch die Kraftkompensierungsanordnung (34) mit einer Kraft entsprechend der erwähnte Seitenkraft kompensiert.

Aufgrund des konstruktiven Aufbaus der Kraftkompensierungsanordnung (34) wirkt diese in der gleichen Weise unabhängig von dem Winkel, den die Strebe (19) mit den Schienen (41,42) bildet. Die Schienen (41,42) sind so angeordnet, daß die Strebe (19) diese passieren kann, was mit der strichpunktierten Lage der Strebe (19) dargestellt ist. Die Arme (35, 36) werden bei einer derartigen Bewegung um den Gelenkarm (40) gedreht.

Im Rahmen des Erfindungsgedanken kann das Parallelogrammsystem außer Armen und Streben aus weiteren konstruktiven Mitteln gebildet werden.

### Bezugszeichenliste

- 1: mobiles Röntgengerat
- 2: Wagen
- 3: Laufrad
- 4: Lenkrad
- 5: Boden
- 6: Ausleger
- 7,8: Tragarm
- 9,10: Welle
- 11: Röntgenrohre
- 12,13: Hebelarm
- 14: Parallelogrammsystem
- 15,16,17,18: Verbindungsknoten
- 19: Strebe
- 20: Kurvenscheibe, keilförmiger Teil
- 21: Rolle
- 22: Feder
- 23: Befestigungspunkt
- 24: Welle
- 25: Stange
- 26: Befestigungspunkt
- 27,28: Oberfläche
- 29: Rolle
- 30: Welle
- 31: Längsstrebe
- 32: Querstrebe
- 33: Welle
- 34: Kraftkompensierungsanordnung
- 35,36: Arm, Gelenkarmanordnung
- 37,38: Welle
- 39: Hülse
- 40: Gelenkachse
- 41,42: Schiene
- 43: Druckfeder
- 44: das eine Ende der Druckfeder
- 45: das andere Ende der Druckfeder

## Patentansprüche

1. Gewichtsausgleichsvorrichtung für eine zweiteilige Tragarmvorrichtung bei einem Röntgengerät (1), mit einem ersten und einem zweiten Tragarm (7,8), bei dem das eine Ende des ersten Tragarms (7) um eine horizontale erste Welle (9) am Röntgengerät (1) drehbar gelagert ist und das andere Ende über eine zweite Welle (10) mit dem einen Ende des zweiten Tragarms (8), an dessen anderem Ende ein Apparat (11) befestigt ist, drehbar verbunden ist, und daß jeder Tragarm (7,8) mit jeweils einem Hebelarm (12,13) versehen ist, der Teil eines Parallelogrammsystems ist, dessen Verbindungsknoten (15,16,17,18) teils am freien Ende des Hebelarms (12) am ersten Tragarm (7), teils an der zweiten Welle (10) und teils am freien Ende des Hebelarms (13) des zweiten Tragarms (8) angeordnet sind, wobei der im Parallelogrammsystem übrige Verbindungsknoten (18) durch eine Kraft beeinflußt wird, die als Gewichtsausgleich für die Tragarmvorrichtung (7,8) um die erste Welle (9) dient, so daß sich die Tragarmvorrichtung in jeder Lage im Gleichgewicht befindet und daß die jeweiligen Längen der Hebelarme (12,13) so gewählt sind, daß die Kraft, die als Gleichgewicht für die Tragarmvorrichtung (7,8) dient, unabhängig von der Lage der Tragarmvorrichtung (7,8) konstant ist, **gekennzeichnet durch** eine Feder (22), deren Kraft ein keilförmiges Teil (20) beeinflußt, welches mit dem übrigen Verbindungsknoten (18) verbunden und hauptsächlich in vertikaler Richtung bewegbar ist und daß die Oberfläche (27) des keilförmigen Teiles (20), die durch die Federkraft beeinflußt wird, eine derartige Neigung aufweist, daß eine Teilkraft entsprechend der konstanten Kraft entsteht.

2. Gewichtsausgleichsvorrichtung nach Anspruch 1, **dadurch gekennzeichnet,** daß die Neigung entlang der Oberfläche (27) in vertikaler Richtung einer derartigen Kurve folgt, daß die Teilkraft konstant ist, unabhängig von der vertikalen Lage des keilförmigen Teils (20) innerhalb bestimmter Grenzen.

3. Gewichtsausgleichsvorrichtung nach Anspruch 2, **dadurch gekennzeichnet,** daß die Kurve im Anschluß an eine der bestimmten Grenzen so gewählt ist, daß die Teilkraft geringer als die konstante Kraft ist.

4. Gewichtsausgleichsvorrichtung nach Ansprüch 1 bis 3, **dadurch gekennzeichnet,** daß die Feder (22) eine Druckfeder ist, deren eines Ende am Röntgengerät (1) befestigt und an derem anderen Ende eine Rolle (21) drehbar angeordnet ist, die gegen die Oberfläche (27) des keilförmigen Teils etwa in horizontaler Richtung drückt.

5. Gewichtsausgleichsvorrichtung nach einem der Ansprüche 1 bis 4, **dadurch gekennzeichnet,** daß das keilförmige Teil (20) mit dem übrigen Verbindungsknoten (18) über eine Strebe (19) verbunden ist.

6. Gewichtsausgleichsvorrichtung nach Anspruch 5, **gekennzeichnet durch** eine zweiteilige Gelenkarmanordnung (35,36), bei der das freie Ende des einen Arms (35) mit der Strebe (19) schwenkbar verbunden ist und bei der das freie Ende des zweiten Arms (36) mit der Strebe (19) in deren axialer Richtung verschiebbar verbunden ist und bei der die Gelenkachse (40) zwischen den Armen (35,36) in einem mit dem Röntgengerät (1) fest verbundenen Teil derart gelagert ist, daß die Gelenkachse (40) lediglich in einer hauptsächlich vertikalen Ebene verschiebbar ist, wobei das freie Ende des zweiten Arms (36) durch eine Kraft in axialer Richtung der Strebe (19) gegen das freie Ende des ersten Arms (35) beeinflußt wird.

7. Gewichtsausgleichsvorrichtung nach Anspruch 6, **dadurch gekennzeichnet,** daß die Kraft durch eine Druckfeder (43) ausgeübt wird, deren eines Ende (44) sich an der Strebe (19) abstützt und deren anderes Ende mit dem freien Ende des zweiten Arms (36) verbunden ist.

8. Gewichtsausgleichsvorrichtung nach Anspruch 6 oder 7, **dadurch gekennzeichnet,** daß der Teil im Röntgengerät (1) aus zwei Schienen (41,42) besteht, zwischen denen die Gelenkachse (40) verläuft.

## Claims

1. Counterbalancing device for a two-part carrying-arm device for an X-ray unit (1), having a first and a second carrying arm (7, 8), in the case of which one end of the first carrying arm (7) is mounted on the X-ray unit (1) such that it can rotate about a horizontal first shaft (9) and the other end is rotatably connected, via a second shaft (10), to one end of the second carrying arm (8), on the other end of which an apparatus (11) is fastened, each carrying arm (7, 8) being provided with in each case one lever arm (12, 13) which is part of a parallelogram system, of which the connection junctions (15, 16, 17, 18) are arranged partly at the free end of the lever arm (12) on the first carrying arm (7), partly on the second shaft (10) and partly at the free end of the lever arm (13) of the second carrying arm (8), the connection junction (18) remaining in the parallelogram system being influenced by a force which serves as counterbalance for the carrying-arm device (7, 8) about the first shaft (9), with the result that the carrying-arm device is in equilibrium in every position, and that the respective lengths of the lever arms (12, 13) are selected such that the force, which serves as equilibrium for the carrying-arm device (7, 8), is constant, irrespective of the position of the carrying-arm device (7, 8), characterized by a spring (22), of which the force influences a wedge-shaped part (20) which is connected to the remaining connection junction (18) and can be moved mainly in the vertical direction, the surface (27) of the wedge-shaped part (20), which surface is influenced by the spring force, being inclined such that a component force corresponding to the constant force results.

2. Counterbalancing device according to Claim 1, characterized in that the inclination along the surface (27) in the vertical direction follows such a curve that the component force is constant, irrespective of the vertical position of the wedge-shaped part (20) within specific limits.

3. Counterbalancing device according to Claim 2, characterized in that, following on from one of the specific limits, the curve is selected such that the component force is smaller than the constant force.

4. Counterbalancing device according to Claims 1 to 3, characterized in that the spring (22) is a compression spring, of which one end is fastened on the X-ray unit (1) and on the other end of which there is rotatably arranged a roller (21) which presses against the surface (27) of the wedge-shaped part approximately in the horizontal direction.

5. Counterbalancing device according to one of Claims 1 to 4, characterized in that the wedge-shaped part (20) is connected to the remaining connection junction (18) via a strut (19).

6. Counterbalancing device according to Claim 5, characterized by a two-part articulation-arm arrangement (35, 36), in the case of which the free end of one arm (35) is pivotably connected to the strut (19), and in the case of which the free end of the second arm (36) is connected to the strut (19) such that it can be displaced in the axial direction of the said strut, and in the case of which the articulation pin (40) between the arms (35, 36) is mounted in a part connected fixedly to the X-ray unit (1), such that the articulation pin (40) can be displaced only in a mainly vertical plane, the free end of the second arm (36) being influenced towards the free end of the first arm (35) by a force in the axial direction of the strut (19).

7. Counterbalancing device according to Claim 6, characterized in that the force is exerted by a compression spring (43), of which one end (44) is supported on the strut (19) and the other end is connected to the free end of the second arm (36)

8. Counterbalancing device according to Claim 6 or 7, characterized in that the part in the X-ray unit (1) comprises two rails (41, 42) between which the articulation pin (40) runs.

## Revendications

1. Dispositif d'équilibrage pour un dispositif en deux parties formant bras de support, dans un appareil radiologique (1), comportant des premier et second bras de support (7,8), une extrémité du premier bras de support (7) étant montée de manière à pouvoir tourner autour d'un premier arbre horizontal (9) de l'appareil radiologique (1) tandis que l'autre extrémité est reliée, de manière à pouvoir tourner, par l'intermédiaire d'un second arbre (10) à une première extrémité du second bras de support (8), sur l'autre extrémité duquel est fixé un appareil (11), et que chaque bras de support (7,8) est muni d'un bras de levier (12,13), qui fait partie d'un système en forme de parallélogramme dont les noeuds de liaison (15,16,17, 18) sont situés en partie sur l'extrémité libre du bras de levier (12) du premier bras de support (7), en partie sur le second arbre (10) et en partie sur l'extrémité libre du bras de levier (13) du second bras de support (8), l'autre noeud de liaison (18), restant du système en forme de parallélogramme étant soumis à une force qui sert de contrepoids au dispositif formant bras de support (7,8) autour du premier arbre (9), de sorte que le dispositif formant bras de support est en équilibre dans toutes les positions, les longueurs des bras de levier (12,13) étant choisies de telle sorte que la force, qui sert de contrepoids au dispositif formant bras de support (7,8), est constante indépendamment de la position du dispositif formant bras de support (7,8), caractérisé par un ressort (22), dont la force s'applique à une pièce en forme de coin (20), qui est reliée noeud de liaison restant et qui est mobile principalement en direction verticale, et que la surface (27) de la pièce en forme de coin (20), qui est soumise à la force du ressort, a une inclinaison telle qu'il se crée une force partielle qui correspond à la force constante.

2. Dispositif d'équilibrage suivant la revendication 1, caractérisé par le fait que l'inclinaison le long de la surface (27) suit, dans la direction verticale, une courbe telle que la force partielle est constante, indépendamment de la position verticale de la pièce en forme de coin (20), dans certaines limites.

3. Dispositif d'équilibrage suivant la revendication 2, caractérisé par le fait que la courbe est choisie au voisinage de l'une des limites déterminées de telle sorte que la force partielle est inférieure à la force constante.

4. Dispositif d'équilibrage suivant l'une des revendications 1 à 3, caractérisé par le fait que le ressort (22) est un ressort de compression, dont une extrémité est fixée à l'appareil radiologique (1) et sur l'autre extrémité duquel est monté rotatif un galet (21), qui s'appuie, sensiblement dans une direction horizontale, contre la surface (27) de la pièce en forme de coin.

5. Dispositif d'équilibrage suivant les revendications 1 à 4, caractérisé par le fait que la pièce en forme de coin (20) est reliée au noeud de liaison (18) restant par l'intermédiaire d'une entretoise (19).

6. Dispositif d'équilibrage suivant la revendication 5, caractérisé par un dispositif à bras articulés (35,36) en deux parties, dans lequel l'extrémité libre de l'un des bras (35) est reliée de manière à pouvoir basculer à l'entretoise (19) et dans lequel l'extrémité libre du second bras (36) est reliée à l'entretoise (19) de manière à pouvoir coulisser dans la direction axiale de celle-ci, et dans lequel l'axe d'articulation (40) entre les bras (35,36) est monté dans une partie solidaire de l'appareil radiologique (1) de telle sorte que l'axe d'articulation (40) ne puisse coulisser que dans un plan principalement vertical, l'extrémité libre du second bras (36) étant repoussée contre l'extrémité libre du premier bras (35) par une force appliquée dans la direction axiale de l'entretoise (19).

7. Dispositif d'équilibrage suivant la revendication 6, caractérisé par le fait que la force est appliquée par un ressort de compression (43), dont l'une des extrémités (44) prend appui sur l'entretoise (19) et dont l'autre extrémité est reliée à l'extrémité libre du second bras (36).

8. Dispositif d'équilibrage suivant la revendication 6 ou 7, caractérisé par le fait que la partie de l'appareil radiologique (1) est formée de deux rails (41,42), entre lesquels s'étend l'axe d'articulation (40).
